# EUROPÄISCHE PATENTSCHRIFT

(11) **EP 3 083 017 B1**
(45) Veröffentlichungstag und Bekanntmachung des Hinweises auf die Patenterteilung: **11.03.2020**
(21) Anmeldenummer: 14828025.8
(22) Anmeldetag: 18.12.2014
(51) Int. Cl.: B01D 61/02, C07C 263/20

(54) **VERFAHREN ZUR ABTRENNUNG VON MONOMEREN ISOCYANATEN**
METHOD FOR SEPARATING ISOCYANATE MONOMERS
PROCÉDÉ POUR SÉPARER DES ISOCYANATES MONOMÈRES

(30) Priorität: 18.12.2013 DE 102013021060
(43) Veröffentlichungstag der Anmeldung: 26.10.2016
(73) Patentinhaber: Sika Technology AG, 6340 Baar (CH)
(72) Erfinder: FRANK, Brian, CH-9000 St. Gallen (CH); KELLENBERGER, Christoph, CH-8006 Zürich (CH)
(74) Vertreter: Sika Patent Attorneys
(86) Internationale Anmeldenummer: PCT/EP2014/078479
(87) Internationale Veröffentlichungsnummer: WO 2015/091807

(56) Entgegenhaltungen:
- EP-A1- 2 042 485
- EP-A1- 2 298 435

## Beschreibung

Die Erfindung betrifft ein Verfahren zur Abtrennung von monomeren Isocyanaten aus Mischungen, die monomere Isocyanate enthalten.

Isocyanathaltige Prepolymere spielen eine große Rolle bei der Herstellung von Montage- und Dämmschäumen, die aus Aerosoldosen ausgebracht werden, um beispielsweise Tür- und Fensterzargen in einem Gebäude einzuschäumen oder Durchbrüche in Mauerwerk zu verschließen. Weitere Einsatzzwecke solcher Prepolymere ist Herstellung von Klebstoffen, insbesondere auch von Sprühklebern.

Solche isocyanathaltigen Prepolymere werden üblicherweise durch Vernetzung von Diphenylmethandiisocyanat (MDI), seinen mehrkernigen Analogen, Toluylendiisocyanat (TDI) und Hexamethylendiisocyanat (HDI) mit Polyolen und/oder Polyesterolen hergestellt. Zahlreiche weitere Isocyanate, die für die Vernetzungsreaktion mit Polyolen oder Polyesterolen eingesetzt werden können, sind bekannt.

Derartig hergestellte Prepolymere enthalten in der Regel mehr oder weniger große Mengen an monomeren Isocyanaten. Diese monomeren Isocyanate haben eine gewisse Flüchtigkeit und sind, in Aerosolform, lungengängig. Gleichzeit sind sie toxisch. Der Gehalt an monomeren Isocyanaten in derartigen Prepolymeren ist deshalb gesetzlichen Einschränkungen unterworfen.

Der Gehalt an monomeren Isocyanaten in derartigen Prepolymeren kann zum einen durch eine geeignete Reaktionsführung und übliche Auswahl der Ausgangsmaterialien bei der Herstellung begrenzt werden. Diese Maßnahmen sind ausgesprochen aufwendig und verteuern das Produkt.

Eine Reihe von monomeren Isocyanaten lässt sich auch durch destillative Maßnahmen entfernen. Eingesetzt wird beispielsweise die Dünnschichtdestillation, auch in Gegenwart eines Schleppmittels, beispielsweise eines hoch siedenden Phosphorsäureesters (TMCP). Solche Phosphorsäureester können in gewissem Umfang im monomerabgereicherten Prepolymer enthalten bleiben, da sie als Flammschutzmittel dienen.

Derartige destillative Verfahren sind relativ einfach durchzuführen, führen aber auch zu einer erheblichen thermischen Belastung des Produkts, das sich hinsichtlich seiner Vernetzungseigenschaften und seiner Viskosität verändert. Dies bringt Einschränkungen bei der Formulierung der fertigen Prepolymermischung für die Aerosoldosen mit sich. Insbesondere müssen Maßnahmen getroffen werden die Löslichkeit des Prepolymers in der Treibgasmischung der Aerosoldose sicherzustellen und die Viskosität auf einen zuträglichen Wert einzustellen.

Angesichts dessen besteht ein Bedarf an neuen Verfahren zur Monomerabreicherung aus isocyanathaltigen Prepolymeren, die den oben genannten Beschränkungen nicht unterliegen, insbesondere zu keiner thermischen Belastung des Prepolymers führen.

Diese Aufgabe wird mit einem Verfahren gelöst, bei dem das Gemisch in einem Lösungsmittel bereitgestellt wird und gegen das Lösungsmittel mittels einer permeablen Membran mit einer Porengröße im Bereich von 5 bis 400 nm dialysiert wird. Bei den Gemischen handelt es sich insbesondere um Prepolymere für die Klebstoff-, Dämm- und Montageschaumherstellung aus Aerosoldosen, die monomere Isocyanate enthalten.

Das erfindungsgemäße Verfahren beruht auf der Abtrennung niedermolekularer Bestandteile aus isocyanathaltigen Prepolymeren mit Hilfe von permeablen Membranen. Bei den niedermolekularen Bestandteilen handelt es sich im Wesentlichen um die Monomere, aus denen die Prepolymere mit Hilfe von vernetzenden Komponenten hergestellt werden. Im Wesentlichen sind dies MDI, TDI und HDI, jedoch kann das Verfahren auch auf andere isocyanatgruppenhaltige Monomere angewandt werden, etwa Isophorondiisocyanat (IPDI) oder Diisocyanatodicyclohexylmethan (H12MDI).

Das Verfahren kann insbesondere auch angewandt werden auf die Abtrennung von monomerem MDI aus Roh-MDI, einer Mischung aus MDI und homologen aromatischen Isocyanaten.

Um die dialytische Abtrennung der Monomeren aus den Prepolymeren zu bewerkstelligen, ist es erforderlich, das Prepolymer in einem Lösungsmittel zu lösen. In Frage kommen im Wesentlichen polare Lösungsmittel oder polare Lösungsmittel enthaltende Mischungen. Geeignete Lösungsmittel sind insbesondere Ether und Ester, aber auch Ketone und halogenhaltige Lösungsmittel.

Unter den Estern sind in erster Linie die Niederalkylester von Ameisensäure und Essigsäure zu nennen, etwa die Methyl- und Ethylester. In Frage kommen auch Phosphorsäureester, beispielsweise Trismonochlorpropylphosphat (TMCP), dessen vollständige Abtrennung aus dem abgereicherten Prepolymer häufig nicht erforderlich ist. TMCP kann in Klebstoffen, Montage- und Dämmschäumen als Flammschutzmittel zum Einsatz kommen.

Zu nennen sind ferner Ether, beispielsweise Dioxolan, THF, Dimethylether und Diethylether, aber auch Methylal. Ein weiterer geeigneter Ether wäre Methyltertiärbutylether. Unter den Ketonen wäre beispielsweise Aceton zu nennen.

Als halogenhaltiges Lösungsmittel kommt beispielsweise Dichlormethan in Frage.

Im Allgemeinen sind Lösungsmittel bevorzugt, die neben guten Lösungseigenschaften einen niedrigen Siedepunkt haben, um eine einfache Abtrennung zu ermöglichen. Bei der Verwendung von Dimethylether wäre das Verfahren wegen des niedrigen Siedepunkts unter Druck durchzuführen. Auch in diesem Fall kann der Ether im abgereicherten Prepolymer (partiell) verbleiben und in der Aerosoldose als Treibgas dienen.

Das in dem Lösungsmittel oder Lösungsmittelgemisch gelöste Prepolymer wird gegen eine permeable Membran dialysiert. Die Membran hat zur Bereitstellung der notwendigen Durchlässigkeit eine Porengröße im Bereich von 5 bis 400 nm, vorzugsweise im Bereich von 15 bis 200 nm und insbesondere von 25 bis 100 nm. Es versteht sich, dass es sich um eine offenporige Membran handelt, um die Durchlässigkeit bei der Dialyse zu gewährleisten.

Die Membran hat im Allgemeinen eine Dicke von 0,05 bis 50 µm, vorzugsweise von 0,5 bis 40 µm und besonders bevorzugt von 5 bis 20 µm. Die Membranstärke wird im Allgemeinen so gewählt, dass die notwendige Stabilität erzielt wird wie auch eine größtmögliche Durchlässigkeit für das jeweilige Monomer. Da die Membran für höher molekulare Bestandteile des Gemisches impermeabel ist, kann sie auch als semipermeabel bezeichnet werden.

Die Membran besteht aus üblichen Kunststoffen, denen auf eine bekannte Art und Weise Porosität verliehen wird. Als Kunststoffe kommen insbesondere in Frage Polysulfone, Polyethersulfone, Polycarbonate, Polyether, Polyester, Polyacrylate, Polysiloxane, Polystyrol wie auch Copolymere davon. Bevorzugt sind Polysulfone, Polyethersulfone und Polyetheretherketone.

Zur Herstellung von Membranen mit geeigneter Porosität wird auf die WO 2012/097967 A1 verwiesen. Dort ist ein Verfahren beschrieben bei dem ein Kunststoff in einem geeigneten Lösungsmittel gelöst wird und diese Lösung mit einer Dispersion von Nanopartikeln eines geeigneten Salzes, etwa Calciumcarbonat, Natriumcarbonat oder Natriumchlorid, versetzt wird. Die Porengröße der permeablen Membran ergibt sich aus der Partikelgröße der Salzteilchen. Nach der Homogenisierung wird die Dispersion/Lösung in der gewünschten Dicke auf einen Träger aufgetragen. Die Salzpartikel werden aus dem Film mit Hilfe einer Säure (Calciumcarbonat) bzw. Wasser (lösliche Salze) herausgewaschen. Der Film wird danach mit Wasser und Ethanol gewaschen, getrocknet und anschließend vom Trägermaterial abgezogen.

Dieses Herstellungsverfahren ist in verschiedenen Varianten mit verschiedenen Techniken eingehend in der WO 2012/097967 A1 beschrieben. Bezüglich der Membranen und ihrer Herstellung wird darauf ausdrücklich Bezug genommen.

Das erfindungsgemäße Verfahren kann bei Normaldruck durchgeführt werden, aber auch unter erhöhtem Druck, um beispielsweise die Dialyse zu fördern oder auch niedrigsiedende Lösungsmittel oder Lösungsmittelgemische einsetzen zu können.

Die Dialyse wird gegen das gleiche Lösungsmittel durchgeführt, in dem auch das Prepolymer gelöst ist. Bevorzugt ist ein Gegenstromverfahren, bei dem die monomerreiche Prepolymerlösung im Gegenstrom zu frischem monomerfreiem Lösungsmittel auf der anderen Seite der Dialysemembran geführt wird. Kontaktzeiten von 0,1 bis 4,0 h sind im Allgemeinen ausreichend. Gute Ergebnisse werden mit Kontaktzeiten von 0,5 bis 1 h erzielt.

Mit dem erfindungsgemäßen Verfahren werden Abreicherungswerte bis in die Gegend der Nachweisgrenze erzielt.

Es versteht sich, dass aus dem monomerabgereicherten Prepolymer das Lösungsmittel zumindest teilweise wieder entfernt werden muss. Dies geschieht durch einfache Destillation, wobei die thermische Belastung des Prepolymers bei niedrig siedenden Lösungsmitteln sehr gering gehalten werden kann. Ggf. kann im Vakuum destilliert werden. Bei der Verwendung von DME als Lösemittel kann dieses durch einfache Drucksenkung auf Normaldruck weitgehend entfernt und zurückgewonnen werden.

Die Erfindung wird durch die nachfolgenden Beispiele näher erläutert.

### Beispiel 1

In einem Dialysevolumen von 2 ml wurde ein MDI-haltiges Prepolymer, gelöst im Verhältnis 1:20 in TMCP, gegen 1000 ml TMCP dialysiert. Als Dialysemembran wurde eine Polyethersulfonmembran mit einer Porengröße von 55 nm eingesetzt. Die Membran hatte eine Dicke von 20 µm.

Das Ausgangsmaterial enthielt 5,8 Gew.-% monomeres MDI.

Nach halbstündiger Dialyse war der Gehalt an monomerem MDI unter die Nachweisgrenze abgesunken.

### Beispiel 2

Eine Lösung eines Prepolymers mit einem Gehalt von 13,2 Gew.-% an monomerem MDI in TMCP 1:20, Dialysevolumen 2 ml, wurde gegen 200 ml TMCP dialysiert. Nach zehnminütiger Dialyse enthielt die Probe noch 7,4 Gew.-% MDI, nach dreißigminütiger Dialyse 2,9 Gew.-%.

Vergleichbare Ergebnisse wurden mit Aceton, THF und Ethylacetat als Lösungsmittel erzielt.

### Beispiel 3

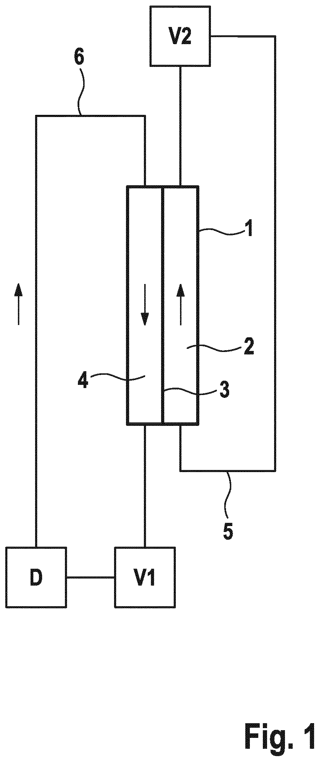

Fig. 1 zeigt eine schematische Darstellung einer Dialyseeinrichtung, wie sie erfindungsgemäß eingesetzt werden kann. In einem Dialysegefäß 1 werden in durch eine permeable Membran 3 getrennten Kanälen 2 und 4 das gelöste Gemisch (Kanal 2) und das Lösungsmittel (Kanal 4) im Gegenstrom geführt. Die monomeren Isocyanate im Kanal 2 gelangen durch die Membran 3 in das Lösungsmittel im Kanal 4 und werden dort abtransportiert.

Die Darstellung zeigt eine diskontinuierliche Verfahrensführung, bei der das Gemisch im Kreis geführt wird (Kanal 2, Leitung 5) und im Sammelbehälter V2 gesammelt wird. Über mehrere Zyklen erfolgt eine Abreicherung des Monomers durch die Membran 3. Im Gegenstrom wird Lösungsmittel über die Leitung 6 und den Kanal 4 geführt. Das mit Monomer beladene Lösungsmittel wird im Sammelbehälter V1 gesammelt. Das Lösungsmittel wird aus der Monomerlösung destillativ abgetrennt (Destillation/Kondensation D) und in den Lösungsmittelstrom zurückgeführt. Das Monomer reichert sich in V1 an.

Das Verfahren kann bei hinreichender Dialyseleistung der Membran auch kontinuierlich geführt werden. In diesem Fall reicht ein Durchlauf durch die Dialyseeinrichtung aus. Die Trennleistung der Einrichtung wird insbesondere bestimmt durch die Membranoberfläche, die Fließgeschwindigkeit der Medien und das Verhältnis von Membranfläche zu anstehendem Volumen des Mediums.

## Patentansprüche

1. Verfahren zur Abtrennung von monomeren Isocyanaten aus isocyanathaltigen Prepolymeren, durch
Bereitstellen des Gemisches in Aceton, TMCP oder Dimethylether als Lösungsmittel und
Dialysieren des gelösten Gemisches gegen das Lösungsmittel mittels einer permeablen Membran aus Polyethersulfon oder Polyetheretherketon mit einer Porengröße im Bereich von 5 bis 400 nm.

2. Verfahren nach Anspruch 1, **dadurch gekennzeichnet, dass** die Membran eine Porengröße im Bereich von 15 bis 200 nm hat, vorzugsweise im Bereich von 25 bis 100 nm.

3. Verfahren nach einem der vorstehenden Ansprüche, **dadurch gekennzeichnet, dass** eine Membran einer Dicke von 0,05 bis 50 µm, vorzugsweise von 0,5 bis 40 µm und besonders bevorzugt von 5 bis 20 µm verwandt wird.

4. Verfahren nach einem der vorstehenden Ansprüche, **dadurch gekennzeichnet, dass** es auf die Abtrennung von monomeren MDI, TDI oder HDI aus Prepolymeren angewandt wird.

5. Verfahren nach einem der vorstehenden Ansprüche, **dadurch gekennzeichnet, dass** es im Gegenstrom geführt wird.

6. Verfahren nach einem der vorstehenden Ansprüche, **dadurch gekennzeichnet, dass** die Konzentration des Gemisches in dem Lösungsmittel im Bereich von 2 bis 20 Gew.-% liegt.

7. Verfahren nach Anspruch 6, **dadurch gekennzeichnet, dass** die Konzentration des Gemisches im Lösungsmittel im Bereich von 4 bis 10 Gew.-% liegt.

8. Verfahren nach einem der vorstehenden Ansprüche, **gekennzeichnet durch** eine Dialysezeit von 0,1 bis 4,0 h.

## Claims

1. Process for removal of monomeric isocyanates from isocyanate-containing prepolymers by providing the mixture in acetone, TMCP or dimethyl ether as solvent and
dialyzing the dissolved mixture against the solvent using a permeable membrane made of polyethersulfone or polyether ether ketone having a pore size in the range from 5 to 400 nm.

2. Process according to Claim 1, **characterized in that** the membrane has a pore size in the range from 15 to 200 nm, preferably in the range from 25 to 100 nm.

3. Process according to either of the preceding claims, **characterized in that** a membrane having a thickness of 0.05 to 50 µm, preferably of 0.5 to 40 µm and particularly preferably of 5 to 20 µm is used.

4. Process according to any of the preceding claims, **characterized in that** it is used for the removal of monomeric MDI, TDI or HDI from prepolymers.

5. Process according to any of the preceding claims, **characterized in that** it is run in countercurrent.

6. Process according to any of the preceding claims, **characterized in that** the concentration of the mixture in the solvent is in the range from 2% to 20% by weight.

7. Process according to Claim 6, **characterized in that** the concentration of the mixture in the solvent is in the range from 4% to 10% by weight.

8. Process according to any of the preceding claims, **characterized by** a dialysis of time of 0.1 to 4.0 h.

## Revendications

1. Procédé pour la séparation d'isocyanates monomériques à partir de prépolymères contenant des isocyanates, par
la mise à disposition du mélange dans de l'acétone, du TMCP ou du diméthyléther en tant que solvant et
la dialyse du mélange dissous contre le solvant à l'aide d'une membrane perméable composée de polyéthersulfone ou de polyétheréthercétone dotée d'une grosseur de pore dans la plage de 5 à 400 nm.

2. Procédé selon la revendication 1, **caractérisé en ce que** la membrane possède une grosseur de pore dans la plage de 15 à 200 nm, de préférence dans la plage de 25 à 100 nm.

3. Procédé selon l'une quelconque des revendications précédentes, **caractérisé en ce qu'**une membrane d'une épaisseur de 0,05 à 50 µm, de préférence de 0,5 à 40 µm et particulièrement préférablement de 5 à 20 µm est employée.

4. Procédé selon l'une quelconque des revendications précédentes, **caractérisé en ce qu'**il est appliqué à la séparation de MDI, de TDI ou de HDI monomérique à partir de prépolymères.

5. Procédé selon l'une quelconque des revendications précédentes, **caractérisé en ce qu'**il est conduit à contre-courant.

6. Procédé selon l'une quelconque des revendications précédentes, **caractérisé en ce que** la concentration du mélange dans le solvant se situe dans la plage de 2 à 20 % en poids.

7. Procédé selon la revendication 6, **caractérisé en ce que** la concentration du mélange dans le solvant se situe dans la plage de 4 à 10 % en poids.

8. Procédé selon l'une quelconque des revendications précédentes, **caractérisé par** un temps de dialyse de 0,1 à 4,0 h.
